# EUROPÄISCHE PATENTSCHRIFT

(11) **EP 1 321 099 B1**
(45) Veröffentlichungstag und Bekanntmachung des Hinweises auf die Patenterteilung: **07.09.2005**
(21) Anmeldenummer: 02102757.8
(22) Anmeldetag: 13.12.2002
(51) Int. Cl.: A61B 6/00, H05G 1/46, H05G 1/30

(54) **Röntgeneinrichtung mit einer Speicheranordnung für Aufnahmeparameter von Röntgenaufnahmen**
X-ray apparatus with memory means to store take parameters of the radiographs
Appareil à rayons X avec mémoire pour stocker les paramètres de prise de vue des radiographies

(30) Priorität: 15.12.2001 DE 10161708
(43) Veröffentlichungstag der Anmeldung: 25.06.2003
(73) Patentinhaber: Philips Intellectual Property & Standards GmbH, 20099 Hamburg (DE); Koninklijke Philips Electronics N.V., 5621 BA Eindhoven (NL)
(72) Erfinder: Maack, Hanns-Ingo, 52088, Aachen (DE); Neitzel, Ulrich, Dr., 52088, Aachen (DE); Günther-Kohlfahl, Susanne, 52088, Aachen (DE)
(74) Vertreter: Volmer, Georg

(56) Entgegenhaltungen:
- DE-A- 10 019 242
- US-A- 5 588 036
- US-A- 5 737 386

## Beschreibung

Die Erfindung betrifft eine Röntgeneinrichtung mit einer Speicheranordnung zum Speichern einer Anzahl von Aufnahmeparameter-Sätzen, von denen jeweils einer für eine nachfolgende Röntgenaufnahme aufrufbar ist. Die Verwendung einer Speicheranordnung, in der für verschiedene Organe je ein Satz von Aufnahmeparametern für eine Röntgenaufnahme gespeichert ist, ist in der Röntgentechnik seit langem bekannt.

Bei dieser sog. APR-Aufnahmetechnik (APR=Anatomically Programmed Radiography) wurden zunächst Aufnahmeparameter für den Röntgengenerator gespeichert, wie etwa die Spannung an der Röntgenröhre, der Strom durch die Röntgenröhre oder das mAs-Produkt. Mit dem Aufkommen so genannter digitaler Bilddetektoren sind Aufnahmeparameter hinzugekommen, die nicht auf Einstellung des Röntgengenerators abzielen, z. B. die Größe des auf dem digitalen Bilddetektors zu belichtenden Aufnahmefeldes oder Bildverarbeitungsparameter.

Der Vorteil einer solchen Röntgeneinrichtung, die aus der US-PS 6,259,767 (PHD 98-124) bekannt ist, besteht darin, dass der Benutzer bei einer Röntgenaufnahme des betreffenden Organs lediglich den diesem Organ zugeordneten Aufnahmeparameter-Satz aufrufen muss, wonach die darin enthaltenen Aufnahmeparameter mit Hilfe geeigneter Einstellmittel automatisch eingestellt werden.

Der Benutzer kann die aufgerufenen Aufnahmeparameter ändern, wenn die Bedingungen bei der Röntgenaufnahme von der üblichen Aufnahmesituation abweichen. Beim nächsten Aufruf dieses Aufnahmeparameter-Satzes werden aber wiederum die ursprünglichen Aufnahmeparameter vorgegeben. Wenn die - in der Regel vom Hersteller der Röntgeneinrichtung vorgegebenen - Aufnahmeparameter nach Meinung des Benutzers grundsätzlich nicht optimal sind, muss er die Aufnahmeparameter bei jedem Aufruf des betreffenden Aufnahmeparameter-Satzes ändern.

Dokument US-5588036 beschreibt eine Röntgeneinrichtung mit einer Speicheranordnung in der die wirksamen Aufnahmeparameter einer vorangegangenen Röntgenaufnahme gespeichert werden. Diese Aufnahmeparameter konnen bei einer erneuten Aufnahme obgeändert werden, wobei jedoch die vorangegangenen Aufnahmeparameter über schrieben werden.

Um diese ständige Änderung zu vermeiden, können die gespeicherten Aufnahmeparameter-Sätze durch den Service des Herstellers oder - dies ist nur für gewisse Aufnahmeparameter möglich - durch den Benutzer geändert werden. Bei der Vielzahl von Aufnahmeparameter-Sätzen - moderne Röntgeneinrichtungen erlauben die Speicherung von nmd 1.000 verschiedenen Aufnahmeparameter-Sätzen - ist dies aber recht aufwendig.

Aufgabe der vorliegenden Erfindung ist es, eine Röntgeneinrichtung zu schaffen, deren Bedienung noch weiter vereinfacht ist. Diese Aufgabe wird gelöst durch eine Röntgeneinrichtung mit
- einer ersten Speicheranordnung zum Speichern einer Anzahl von ersten Aufnahmeparameter-Sätzen, von denen jeweils einer für eine nachfolgende Röntgenaufnahme aufrufbar ist,
- Mitteln zur Änderung der aufgerufenen Aufnahmeparameter,
- einer zweiten Speicheranordnung, in der eine Anzahl von zweiten Aufnahmeparameter-Sätzen speicherbar ist, von denen jeder die nach einem Aufruf eines ersten Aufnahmeparameter-Satzes bei der nachfolgenden Röntgenaufnahme tatsächlich wirksamen Aufnahmeparameter oder daraus abgeleitete Größen umfaßt,
- Mitteln zum Auswerten der zweiten Aufnahmeparameter-Sätze, die demselben ersten Aufnahmeparameter-Satz zugeordnet sind, zur Ableitung eines neuen Aufnahmeparameter-Satzes,
- Mitteln zum Speichern des neuen Aufnahmeparameter-Satzes anstelle des ersten Aufnahmeparameter-Satzes in der ersten Speicheranordnung,

Bei der Erfindung werden bei einem Aufruf eines in der ersten Speicheranordnung gespeicherten Aufnahmeparameter-Satzes die bei der nachfolgenden Röntgenaufnahme tatsächlich wirksamen Aufnahmeparameter (oder daraus abgeleitete Größen) in einer zweiten Speicheranordnung gespeichert. Wenn ein Aufnahmeparameter-Satz aus der ersten Speicheranordnung genügend oft aufgerufen worden ist, ist in der zweiten Speicheranordnung eine Anzahl von Aufnahmeparameter-Sätzen gespeichert, die die bei den Röntgenaufnahmen tatsächlich wirksamen Aufnahmeparameter enthalten.

Durch die Auswertung werden die Unterschiede zwischen einem der aus der ersten Speicheranordnung aufgerufenen Aufnahmeparameter eines Satzes und der Größe dieses Aufnahmeparameters bei den nachfolgenden Röntgenaufnahmen erkennbar, sodass sich ein neuer Aufnahmeparameter ermitteln läßt mit einer bei den vorherigen Röntgenaufnahmen tatsächlich wirksamen Größe. Werden die Aufnahmeparameter der in der ersten Speicheranordnung gespeicherten Sätze durch diese neuen Aufnahmeparameter ersetzt, dann ergeben sich Aufnahmeparameter-Sätze, die bei künftigen Röntgenaufnahmen voraussichtlich weniger oft geändert werden müssen. Die Röntgeneinrichtung "lernt" auf diese Weise also die für ihren jeweiliges Benutzerkreis optimalen Aufnahmeparameter. Weil an verschiedenen Röntgeninstituten oder Kliniken oft unterschiedliche Einstelltechniken üblich sind, können sich diese neuen Aufnahmeparameter-Sätze von Institut zu Institut unterscheiden.

Es sei an dieser Stelle bemerkt, dass bei manchen Röntgeneinrichtungen bereits eine Vielzahl von technischen Parametern gespeichert werden, darunter die Aufnahmeparameter - sowohl bei APR-Technik als auch bei freier Einstelltechnik. Auf diese Daten hat in der Regel aber nur der Hersteller einen Zugriff, und sie dienen nicht unmittelbar der Einstellung der betreffenden Röntgeneinrichtung.

In der Regel kann davon ausgegangen werden, dass ein aus der ersten Speicheranordnung aufgerufener und gegebenenfalls vom Benutzer modifizierter Aufnahmeparameter bei der Aufnahme auch tatsächlich wirksam ist, beispielsweise die Röhrenspannung. Dies gilt aber nicht für alle Aufnahmeparameter. Ein bei digitalen Detektoren benutzter Aufnahmeparameter ist beispielsweise der so genannte Exposure Index, der mit der Empfindlichkeit der früher für Röntgenaufnahmen benutzten Film-Folien-Kombinationen korrespondiert und aus dem die Dosis ableitbar ist, die für eine optimale Belichtung benötigt wird. Führt man eine solche Röntgenaufnahme mit einem bestimmten mAs-Produkt als Aufnahmeparameter aus, dann hängt es von der Strahlenabsorption durch den jeweiligen Patienten ab, ob die Röntgenaufnahme tatsächlich entsprechend dem vorgegebenen Exposure Index belichtet wird oder nicht. Der Exposure Index kann nicht direkt gemessen werden, sondern eine daraus abgeleitete Größe, nämlich die Dosis am Bildempfänger - und dies auch erst nach einer Röntgenaufnahme. Eine für diesen Fall geeignete Ausgestaltung der Erfindung ist in Anspruch 2 definiert.

Bei der Erfindung erfolgt nicht nach jeder Röntgenaufnahme eine Auswertung der Aufnahmeparameter zur Ableitung neuer Aufnahmeparameter, die dann in die erste Speicheranordnung übernommen werden. Gemäß Anspruch 3 erfolgt dies erst nach Erfüllung eines Aktivierungskriteriums. Dabei kann es sich z B. um die Zahl der seit der letzten Aktivierung angefertigten Röntgenaufnahmen oder - für einen einzelnen Aufnahmeparameter-Satz-um eine bestimmte Zahl von Aufrufen dieses Aufnahmeparameter-Satzes handeln oder aber um einen bestimmter Zeitraum, der seit der letzten Aktivierung verstrichen ist. Bei der Ausgestaltung nach Anspruch 4 hat demgegenüber Benutzer oder der Service es in der Hand, diesen Vorgang durch Aktivierung des Anpassungsmodus einzuleiten.

Die Ausgestaltung nach Anspruch 5 hat den Sinn zufällige oder zu häufige Änderungen der Aufnahmeparameter zu vermeiden bzw. unsinnige Einstellungen auszuschließen. Deshalb werden die aus der Auswertung der zweiten Aufnahmeparameter-Sätze abgeleiteten Aufnahmeparameter erst nach einer Überprüfung in die erste Speicheranordnung übernommen.

Die Erfindung wird nachstehen an Hand eines Ablaufdiagramms näher erläutert. Es zeigen
- Fig. 1: ein schematisches Blockschaltbild einer Röntgeneinrichtung.
- Fig. 2: ein Ablaufdiagramm des mit einer solchen Röntgeneinrichtung durchführbaren Aufnahmemodus und
- Fig. 3: ein Ablaufdiagramm des mit der Röntgeneinrichtung durchführbaren Anpassungsmodus.

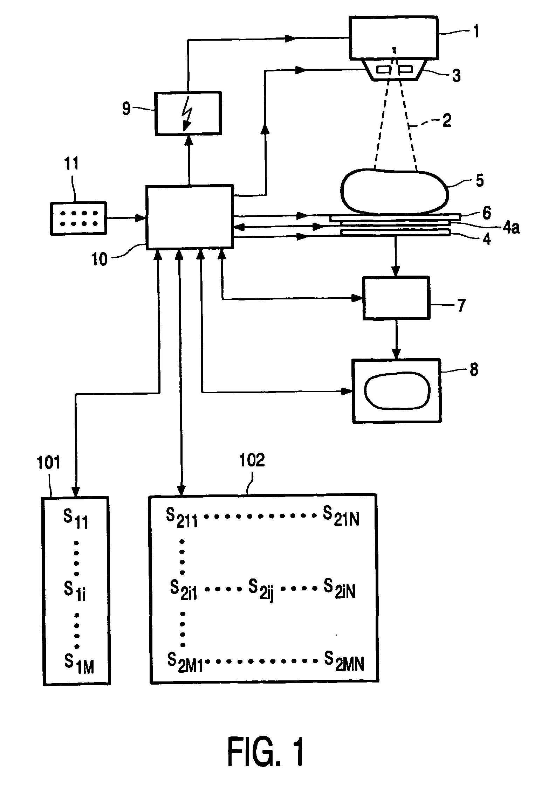

Die in Fig 1 dargestellte Röntgeneinrichtung umfasst einen Röntgenstrahler 1, der während einer Röntgenaufnahme ein Strahlenbündel 2 emittiert, dessen Querschnitt durch eine steuerbare Tiefenblende 3 veränderbar ist. Das Strahlenbündel 2 trifft auf einen Bildempfänger 4, nachdem es ein Untersuchungsobjekt 5 durchsetzt hat, das sich auf der Lagerungsplatte 6 eines im Übrigen nicht dargestellten Untersuchungstisches befindet.

Zwischen dem Bildempfänger 4 und der Lagerungsplatte 6 befindet sich ein Sensor 4a der Belichtungsautomatik, der die Dosis hinter dem Untersuchungsobjekt 5 mißt.

Obwohl die Erfindung auch bei konventionellen Film-Folien-Kombinationen als Bildempfänger anwendbar ist, wird im Folgenden davon ausgegangen, dass es sich bei dem Bildempfänger 4 um einen so genannten digitalen Bilddetektor handelt, d. h um einen Detektor, der elektrisch auslesbar ist und Bildsignale liefert, die von der Strahlendosis an den verschiedenen Punkten (Pixeln) des Detektors abhängig sind Aus diesen Bildsignalen wird in einer Bildverarbeitungseinheit 7 eine Röntgenaufnahme rekonstruiert und auf einem Monitor 8 wiedergegeben. Die Leistungsversorgung des Röntgenstrahlers 1 erfolgt durch einen Röntgengenerator 9.

Die Komponenten 1 ... 9 werden von einer Steuereinheit 10, beispielsweise einer Workstation, gesteuert, auf die der Benutzer über eine Eingabeeinheit 11 zugreifen kann. Die vom Benutzer eingegebenen oder durch seine Eingabe aufgerufenen Daten können auf dem Monitor 8 oder aber einem gesonderten Display wiedergegeben werden.

Die für die Erstellung einer Röntgenaufnahme benötigten Aufnahmeparameter können entweder vom Benutzer frei eingestellt werden, oder der Benutzer ruft bei APR-Röntgenaufnahmen einen Aufnahmeparameter-Satz auf, und die Steuereinheit 10 stellt automatisch die Komponenten 1 ... 9 entsprechend ein. Zu diesem Zweck greift die Steuereinheit 10 auf eine erste Speicheranordnung 101 zu, in der eine Anzahl von ersten Aufnahmeparametersätzen S₁₁ ... S₁ᵢ ... S_{1M} gespeichert sind. M ist dabei die Zahl der ersten Aufnahmeparameter-Sätze, die über 1.000 betragen kann. Jeder Aufnahmeparameter-Satz umfasst eine Anzahl L von Aufnahmeparametern.

Diese sind teilweise schon vor Beginn einer Röntgenaufnahme eingestellt, wie z. B. die Größe des zu belichtenden Aufnahmefeldes, oder sie werden erst während einer Röntgenaufnahme wirksam, wie z. B. die Spannung an dem Röntgenstrahler, oder sie ergeben sich erst bei Aufnahmeende, wie z. B. die Aufnahmedauer oder die Dosis am Bildempfänger, die auch erst nach der Röntgenaufnahme bestimmt werden kann. Manche Parameter können unmittelbar so eingestellt werden, wie sie vorgegeben werden, z B. die Spannung am Röntgenstrahler (wenn man einmal von Einstellfehlern absieht). Andere Parameter, wie z B. die Dosis am Bildempfänger 4 oder der dazu inverse Exposure Index, können nicht unmittelbar eingestellt werden, doch werden sie durch andere Aufnahmeparameter beeinflusst, wie z B. das mAs-Produkt.

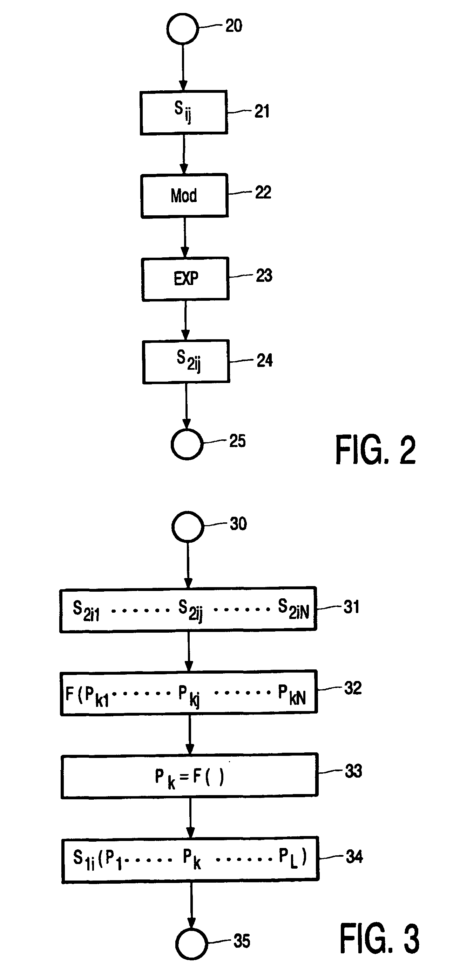

Im Folgenden wird an Hand des Ablaufdiagramms in Fig 2 die Funktion der Röntgeneinrichtung im Aufnahmemodus beschrieben, in dem Röntgenaufnahmen erstellt werden. Nach der Initialisierung in Schritt 20 ruft der Benutzer im Schritt 21 über die Eingabeeinheit 11 einen ersten Aufnahmeparameter-Satz S₁ᵢ auf, wobei i zwischen 1 und M liegt. Die zu diesem Satz gehörenden Aufnahmeparameter werden dem Benutzer z. B. auf dem Monitor 8 angezeigt und können von ihm im Schritt 22 mittels der Eingabeeinheit 11 modifiziert werden. Mit diesen vorgegebenen und gegebenenfalls modifizierten und über geeignete Stellglieder in der Röntgeneinrichtung eingestellten Aufnahmeparametern wird im Schritt 23 eine Röntgenaufnahme erstellt.

Im Schritt 24 werden dann die Aufnahmeparameter gespeichert, die während der Aufnahme wirksam waren. Es handelt sich dabei um Aufnahmeparameter, die bereits in dem aufgerufenen Parametersatz enthalten waren oder um geänderte Aufnahmeparameter. Einige Aufnahmeparameter, wie z. B. der Exposure Index oder die Dosis am Bildempfänger, können erst nach einer Röntgenaufnahme bestimmt werden (indem zwecks Bildung eines Mittelwertes über die von dem Bildempfänger 4 gelieferten Bildsignale integriert wird).

Die während der Röntgenaufnahme wirksamen Aufnahmeparameter bilden einen zweiten Aufnahmeparameter-Satz S₂ᵢⱼ, wobei der Index i diesen zweiten Aufnahmeparameter-Satz mit dem ersten Aufnahmeparameter-Satz S₁ᵢ verknüpft, und der Index j einen von der Zahl der Aufrufe des ersten Aufnahmeparameter-Satzes S₁ᵢ abhängigen Wert hat. Dieser zweite Aufnahmeparameter-Satz wird in einer zweiten Speicheranordnung 102 gespeichert, die für jeden ersten Aufnahmeparameter-Satz N Aufnahmeparameter-Sätze speichern kann, sodass sich für den ersten Aufnahmeparameter-Satz S₁ᵢ in diesem Speicher die Aufnahmeparameter-Sätze S₂ᵢₗ ... S₂ᵢ... S_{2jN} ergeben. Damit ist für die betreffende Röntgenaufnahme der Aufnahmemodus beendet (Schritt 25).

Zu entweder frei wählbaren Zeitpunkten oder automatisch nach einer vorbestimmten Anzahl von Röntgenaufnahmen oder nach Ablauf einer bestimmten Zeit erfolgt ein Abgleich zwischen den in den beiden Speicheranordnungen 101 und 102 gespeicherten Aufnahmeparameter-Sätzen. Darm arbeitet die Röntgeneinrichtung in einem Anpassungsmodus, dessen Ablaufdiagramm in Fig 3 dargestellt ist. Dazu werden im Schritt 31 die Aufnahmeparameter-Sätze S₂ᵢ₁ ... S_{2iN} aufgerufen, die bei den APR-Röntgenaufnahmen wirksam waren, für die aus der ersten Speichenanordnung der Aufnahmeparameter-Satz S₁ᵢ aufgerufen wurde.

Für jeden Aufnahmeparameter Pₖ des ersten Aufnahmeparameter-Satzes - z B. das mAs-Produkt - werden im Schritt 32 die während der N Röntgenaufnahmen wirksamen Werte Pₖₗ ... P_{kN} analysiert und mit dem im ersten Aufnahmeparameter-Satz enthaltenen Parameter Pₖ verglichen, indem beispielsweise ihr arithmetischer Mittelwert oder der Medianwert gebildet wird. Im Einzelnen kann diese Auswertung der Aufnahmeparameter von der Natur der Aufnahmeparameter abhängen. Während es für kontinuierlich variierbare Aufnahmeparameter wie das mAs-Produkt oder die Größe eines Aufnahmefeldes beispielsweise sinnvoll sein kann, aus dem bei der Röntgenaufnahme wirksamen Aufnahmeparametern einen arithmetischen Mittelwert oder einen Medianwert abzuleiten, ist es für andere Aufnahmeparameter, z B. das Vorhandensein oder Nichtvorhandensein eines Streustrahlenrasters im Strahlengang, sinnvoll, die weiteren Vorgaben danach auszurichten, welche der beiden Möglichkeiten (mit Streustrahlenraster oder ohne Streustrahlenraster) bei den N Aufnahmen häufiger gewählt wurde.

Bevor den Aufnahmeparametern des Aufnahmeparameter-Satzes S₁ᵢ die auf diese Weise aus den vorangegangenen Röntgenaufnahmen ermittelten Aufnahmeparameter Pₖ zu Grunde gelegt werden, ist es sinnvoll, im Schritt 33 zunächst weitere Überprüfungen vorzunehmen. Um zufällige oder zu häufige Änderungen der Aufnahmeparameter zu vermeiden, können Schwellen für die Anpassung vorgesehen sein, sodass ein bisher im ersten Speicher enthaltener Aufnahmeparameter erhalten bleibt, wenn er nur relativ selten verändert wurde. Wenn er dagegen relativ häufig und in derselben Richtung geändert wurden, z B. wenn das Aufnahmefeld in mehr als 50 Prozent der Fälle vergrößert wurde, wird der betreffende Aufnahmeparameter entsprechend geändert. Weiterhin kann es zweckmäßig sein, den Wertebereich für die Aufnahmeparameter so einzuschränken, dass unsinnige Einstellungen ausgeschlossen werden. Danach kann im Schritt 33 dem betreffenden Parameter Pₖ ein entsprechender Wert zugewiesen werden. Dies wird für alle Aufnahmeparameter P₁ ... Pₖ ... P_{L} eines Satzes wiederholt, sodass danach ein neuer Satz S₁ᵢ von (zumindest teilweise geänderten) Aufnahmeparametern vorhanden ist, der an Stelle des bisherigen Satzes in der ersten Speicheranordnung 101 gespeichert wird (Schritt 34).

Die Schritte 31...34 können für andere von Aufnahmeparameter-Sätze wiederholt werden, wonach der Anpassungsmodus beendet wird (Schritt 35). Der Anpassungsmodus kann aber auch automatisch aktiviert werden, wenn für einen einzelnen Aufnahmeparameter-Satz ein Aktivierungskriterium erfüllt ist, beispielsweise, wenn seit der letzten Anpassung eine bestimmte Zahl von Röntgenaufnahmen unter Aufruf dieses Satzes erstellt wurde.

Wie bereits ausgeführt, werden bei bestimmten Aufnahmearten (ohne Belichtungsautomatik) ein mAs-Produkt und ein bestimmter Exposure Index bzw. eine bestimmte Dosis am Bildempfänger als Aufnahmeparameter vorgegeben. Diese Aufnahmeparameter sind voneinander abhängig. In diesem Fall wird in den Schritten 31...34 aus den nach den Aufnahmen ermittelten Werten des Exposure Index bzw. aus der Dosis am Bildempfänger nicht ein neuer Wert dieses Aufnahmeparameters ermittelt, sondern diese Werte werden benutzt, um einen besser geeigneten Wert des mAs-Produktes zu bestimmen. Ergibt sich bei der Auswertung im Schritt 32 bzw. 33 beispielweise, dass die Empfängerdosis bei den vorangegangenen Aufnahmen im Mittel um einen Faktor 2 zu hoch (bzw. der Exposure Index um einen Faktor 2 zu niedrig) war, dann wird das bei den Aufnahmen eingestellte mAs-Produkt um den Faktor 2 erniedrigt und als neuer Aufnahmeparameter vorgegeben.

Daneben gibt es auch Aufnahmen mit Belichtungsautomatik, bei denen eben dem Exposure Index eine Empfindlichkeitsstufe der Belichtungsautomatik vorgegeben ist, von der erwartet wird, dass die Detektordosis typischerweise einen bestimmten Wert erreicht, der einer bestimmten Empfindlichkeitsklasse zugeordnet wird (zu den Vorgaben der Empfindlichkeitsklassen gibt es ärztliche Standards, zB. die Ärztlichen Leitlinien). Diese Empfindlichkeitsstufe kann ähnlich wie der mAs Wert angepasst werden. Ergibt sich bei der Auswertung im Schritt 32 bzw. 33 beispielweise, dass die Empfängerdosis bei den vorangegangenen N Aufnahmen im Mittel um einen Faktor 2 zu hoch (bzw. der Exposure Index um einen Faktor 2 zu niedrig) war, dann wird der bei den Aufnahmen eingestellte Empfindlichkeitsstufe um 3 Stufen (entsprechend einem Faktor 2) erniedrigt und als neuer Aufnahmeparameter vorgegeben.

Es ist nicht erforderlich, dass für die Speicherung der ersten und zweiten Aufnahmeparameter-Sätze getrennte Speicheranordnungen 101 und 102 benutzt werden. Die Aufnahmeparameter können in dem selben Speicher gespeichert werden. Die aus den Röntgenaufnahmen resultierenden zweiten Aufnahmeparameter-Sätze müssen auch nicht auf einem bestimmten Speicherplatz gespeichert sein, sondern können der Reihe nach gespeichert werden, wobei zusätzlich eine Identifizierung für den aufgerufenen ersten Aufnahmeparameter-Satz gespeichert wird.

## Patentansprüche

1. Röntgeneinrichtung mit
- einer ersten Speicheranordnung (101) zum Speichern einer Anzahl von ersten Aufnahmeparameter-Sätzen (S₁₁ ... S₁ᵢ ... S_{1M}), von denen jeweils einer für eine nachfolgende Röntgenaufnahme aufrufbar ist,
- Mitteln (11) zur Änderung der aufgerufenen Aufnahmeparameter (P₁...Pₖ...P_{L}),
- einer zweiten Speicheranordnung (102), in der eine Anzahl von zweiten Aufnahmeparameter-Sätzen S₂₁₁ ... S₂ᵢⱼ ... S_{2MN}) speicherbar ist, von denen jeder die nach einem Aufruf eines ersten Aufnahmeparameter-Satzes bei der nachfolgenden Röntgenaufnahme tatsächlich wirksamen Aufnahmeparameter oder daraus abgeleitete Größen umfaßt,
- Mitteln (10, 31...33) zum Auswerten der zweiten Aufnahmeparameter-Sätze (S₂ᵢ₁...S₂ᵢⱼ...S_{2iN}), die demselben ersten Aufnahmeparameter-Satz (S₁ᵢ) zugeordnet sind zur Ableitung eines neuen Aufnahmeparameter-Satzes,
- Mitteln (10,34) zum Speichern des neuen Aufnahmeparameter-Satzes anstelle des ersten Aufnahmeparameter-Satzes in der ersten Speicheranordnung (101.

2. Röntgeneinrichtung nach Anspruch 1 mit einem elektrisch auslesbaren Bildempfänger (4), der von der empfangenen Strahlendosis abhängige Bildsignale zur Erzeugung einer Röntgenaufnahme liefert und mit Mitteln zum Bestimmen der Strahlendosis aus den Bildsignalen nach einer Röntgenaufnahme, wobei ein Aufnahmeparameter eine mit der der Strahlendosis am Bildempfänger zusammenhängende Größe ist, und Mittel zum Berechnen des Wertes dieses Aufnahmeparameters, der für die Erzielung der gewünschten Strahlendosis erforderlich ist.

3. Röntgeneinrichtung nach Anspruch 1 mit Mitteln zum automatischen Aktivieren der Auswertung der zweiten Aufnahmeparameter-Sätze und der Speicherung von neuen Aufnahmeparameter-Sätzen nach Erfüllung eines Aktivierungskriteriums.

4. Röntgeneinrichtung nach Anspruch 1, die in einem Aufnahmemodus betreibbar ist, in dem bei jeder Röntgenaufnahme die tatsächlich wirksamen Aufnahmeparameter oder daraus abgeleitete Größen gespeichert werden, und das in einem Anpassungsmodus betreibbar ist, bei dessen Aktivierung jeweils die zweiten Aufnahmeparameter ausgewertet und daraus abgeleitete neue Aufnahmeparameter in der ersten Speicheranordnung gespeichert werden.

5. Röntgeneinrichtung nach Anspruch 1 mit Mitteln zum der aus der Auswertung der zweiten Aufnahmeparameter-Sätze (S₂ᵢ₁...S₂ᵢⱼ...S_{2iN}) abgeleiteten Größe eines Aufnahmeparameters vor der Übernahme in die erste Speicheranordnung.

## Claims

1. An X-ray apparatus which includes
- a first storage arrangement (101) for storing a number of first sets of exposure parameters (S₁₁ ... S₁ᵢ ... S_{1M}) of which each time one can be fetched for a subsequent X-ray exposure,
- means (11) for changing the fetched exposure parameters (P₁ ... Pₖ ... P_{L}),
- a second storage arrangement (102) in which a number of second sets of exposure parameters (S₂₁₁ ... S₂ᵢⱼ ... S_{2MN}) can be stored, each of said second sets of exposure parameters containing the exposure parameters, or values derived therefrom, which are actually active during the subsequent X-ray exposure after the fetching of a first set of exposure parameters,
- means (10, 31 ... 33) for evaluating the second sets of exposure parameters (S₂ᵢ₁ ... S₂ᵢⱼ ... S_{2iN}) associated with the same first set of exposure parameters (S₁ᵢ) in order to derive a new set of exposure parameters,
- means (10, 34) for storing the new set of exposure parameters in the first storage arrangement (101) instead of the first set of exposure parameters.

2. An X-ray apparatus as claimed in claim 1, which includes an image receiver (4) which can be read out electrically and supplies image signals which are dependent on the radiation dose received in order to form an X-ray image, and also means for determining the radiation dose from the image signals after an X-ray exposure, an exposure parameter being a variable related to the radiation dose on the image receiver, and also means for calculating the value of this exposure parameter which is necessary for realizing the desired radiation dose.

3. An X-ray apparatus as claimed in claim 1 which includes means for the automatic activation of the evaluation of the second sets of exposure parameters and the storage of new sets of exposure parameters after an activation criterion has been satisfied.

4. An X-ray apparatus as claimed in claim 1, which can be made to operate in an exposure mode in which for each X-ray exposure the actually active exposure parameters, or values derived therefrom, are stored, and also in an adaptation mode in which, when this mode is activated, the second exposure parameters are each time evaluated and new exposure parameters derived therefrom are stored in the first storage arrangement.

5. An X-ray apparatus as claimed in claim 1 which includes means for checking the value of an exposure parameter derived from the evaluation of the second sets of exposure parameters (S₂ᵢ₁ ... S₂ᵢⱼ ... S_{2iN}) before it is stored in the first storage arrangement.

## Revendications

1. Appareil à rayons X avec
- une première mémoire (101) pour l'enregistrement d'un nombre de premiers jeux de paramètres de prise de vue (S₁ₗ...S₁ᵢ...S_{1M}) dont un peut à chaque fois être appelé pour une radiographie suivante;
- des moyens (11) pour modifier les paramètres de prise de vue (P₁...Pₖ...P_{L}) appelés;
- une deuxième mémoire (102) dans laquelle un nombre de deuxièmes jeux de paramètres de prise de vue S₂₁₁ ...S₂ᵢⱼ... S_{2MN}) peut être enregistré, dont chacun comprend les paramètres de prise de vue effectivement actifs lors de la radiographie suivante après la consultation d'un premier jeu de paramètres de prise de vue ou des grandeurs dérivées de ceux-ci,
- des moyens (10...31...33) pour l'évaluation des deuxièmes jeux de paramètres de prise de vue (S₂ᵢ₁...S₂ᵢⱼ...S_{2iN}) qui sont affectés au même premier jeu de paramètres de prise de vue (S₁ᵢ) pour la déduction d'un nouveau jeu de paramètres de prise de vue,
- des moyens (10, 34) pour l'enregistrement du nouveau jeu de paramètres de prise de vue à la place du premier jeu de paramètres de prise de vue dans la première mémoire (101).

2. Appareil à rayons X selon la revendication 1 avec un récepteur d'image (4) lisible électriquement qui délivre des signaux d'image dépendant de la dose de rayons reçue pour la production d'un cliché radiographique et avec des moyens pour la détermination de la dose de rayons à partir des signaux d'image après un cliché radiographique, un paramètre de prise de vue étant une grandeur concordant avec la dose de rayons sur le récepteur d'images et des moyens pour le calcul de la valeur de ce paramètre de prise de vue qui est nécessaire pour atteindre la dose de rayons souhaitée.

3. Appareil à rayons X selon la revendication 1 avec des moyens pour l'activation automatique de l'évaluation des deuxièmes jeux de paramètres de prise de vue et l'enregistrement de nouveaux paramètres de prise de vue après l'exécution d'un critère d'activation.

4. Appareil à rayons X selon la revendication 1 qui peut fonctionner dans un mode de cliché dans lequel, à chaque cliché radiographique, les paramètres de prise de vue effectivement actifs ou les grandeurs dérivées de ceux-ci sont enregistrés et qui peut fonctionner en mode d'adaptation en cas d'activation duquel les deuxièmes paramètres d'évaluation sont respectivement évalués et les nouveaux paramètres de prise de vue dérivés de ceux-ci sont enregistrés dans la première mémoire.

5. Appareils à rayons X selon la revendication 1 avec des moyens pour le contrôle de la grandeur dérivée de l'évaluation des deuxièmes jeux de paramètres de prise de vue (S₂ᵢ₁...S₂ᵢⱼ...S_{2iN}) d'un paramètre de prise de vue avant la réception dans la première mémoire.
